(19) Europäisches Patentamt
European Patent Office
Office européen des brevets

(11) Veröffentlichungsnummer: **0 142 027**
**A1**

# (12) EUROPÄISCHE PATENTANMELDUNG

(21) Anmeldenummer: 84112122.1

(22) Anmeldetag: 10.10.84

(51) Int. Cl.[4]: C 07 D 233/42
C 07 D 233/32, C 07 D 405/06
C 07 D 409/06, C 07 D 409/12
A 61 K 31/415

(30) Priorität: 13.10.83 DE 3337180

(43) Veröffentlichungstag der Anmeldung:
22.05.85 Patentblatt 85/21

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Beck, Gerhard, Dr.
Gustav-Freytag-Strasse 24
D-6000 Frankfurt am Main 1(DE)

(72) Erfinder: Bartmann, Wilhelm, Dr.
Am Dachsbau 5
D-6232 Bad Soden am Taunus(DE)

(72) Erfinder: Lau, Hans-Hermann, Dr.
Kastanienhain 29
D-6232 Bad Soden am Taunus(DE)

(72) Erfinder: Wess, Günther, Dr.
Hainstrasse 35
D-6455 Erlensee(DE)

## (54) Neue 5-substituierte-delta 2-Imidazolinyl-Thioether und Verfahren zu ihrer Herstellung.

(57) Die vorliegende Anmeldung betrifft 5-substituierte-Δ 2-Imidazolinyl-Thioether sowie Zwischenprodukte und Verfahren zu ihrer Herstellung. Die erfindungsgemäßen Verbindungen zeichnen sich insbesondere durch ihre Thrombozytenaggregations-hemmende und blutdrucksenkende Wirkung aus.

EP 0 142 027 A1

Croydon Printing Company Ltd.

## NEUE 5-SUBSTITUIERTE-Δ2-IMIDAZOLINYL-THIOETHER UND VERFAHREN ZU IHRER HERSTELLUNG

Prostacyclin $PGI_2$, ein 1976 isolierter Naturstoff aus der Familie der Prostaglandine, zeichnet sich durch seine stark ausgeprägten thrombocytenaggregationshemmenden Eigenschaften aus (The Lancet 1977, 18). Außerdem vermag $PGI_2$ einige Blutgefäße, z.B. Coronararterien zu relaxieren (Prostaglandins 13, 3, 1977), so daß es zur Therapie und Prophylaxe von Thrombosen und Infarkten Verwendung finden kann. $PGI_2$ zeigt weiter eine ausgeprägte blutdrucksenkende Wirkung (z.B. IRCS Med. Sci. 6, 392 (1978)).

Gegenstand der vorliegenden Erfindung sind neue Verbindungen der allgemeinen Formel I

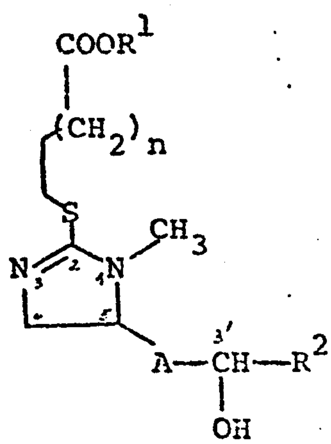

I

die eine spezifischere Wirkung und/oder längere Wirkungsdauer als $PGI_2$ besitzen und in welcher bedeuten:

$R^1$ Wasserstoff, einen geradkettigen oder verzweigten Alkylrest mit bis zu 8 Kohlenstoffatomen, einen geradkettigen oder verzweigten ungesättigten aliphatischen

Kohlenwasserstoffrest mit 3 bis 6 Kohlenstoffatomen, einen cycloaliphatischen Kohlenwasserstoffrest mit 3 bis 7 Kohlenstoffatomen, einen araliphatischen Kohlenwasserstoffrest mit 7 bis 9 Kohlenstoffatomen oder ein physiologisch verträgliches Metall-, $NH_4$- oder ein Ammoniumion, das sich von einem primären, sekundären oder tertiären Amin ableitet, oder ein Tetraalkylammonium

$R^2$ einen Phenylrest, der im Kern 1 - 3-fach substituiert sein kann mit Halogen, Trifluormethyl, und/oder Alkyl oder Alkoxy mit je 1 - 6 C-Atomen, oder einen cycloaliphatischen Rest mit 3 - 8 Kohlenstoffatomen, einen geradkettigen oder verzweigten Alkylrest mit bis zu 8 Kohlenstoffatomen oder einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 6 Kohlenstoffatomen oder einem geradkettigen oder verzweigten Alkenyloxy- oder Alkinyloxy mit 3 bis 6 Kohlenstoffatomen,

b) Halogen, Cycloalkyl mit 3 - 7 Atomen, einem unsubstituierten Phenyl-, α- oder ß-Thienyl- oder α- oder ß-Furylrest oder einem Phenyl-, Thienyl- oder Furylrest, welche ihrerseits im Kern 1 - 3 fach substituiert sind mit Halogen, Trifluormethyl und/oder Alkyl oder Alkoxy mit 1 - 6 C-Atomen,

c) einem unsubstituierten Phenoxy-, α- oder ß-Thienyloxy- oder Cycloalkoxyrest mit 3 - 7 Kohlenstoffatomen, oder einen der genannten Reste, welcher seinerseits im Kern 1 - 3-fach substi-

tuiert ist mit Halogen, Trifluormethyl und/oder Alkyl oder Alkoxy mit je 1 - 6 C-Atomen,

n die Zahl 0, 1, 2, 3 oder 4 und

A eine $-CH=CH-$ oder $-CH_2-CH_2-$Gruppe.

Unter den Substituenten $R^1$ sind bevorzugt:

Wasserstoff, geradkettiges oder verzweigtes Alkyl mit bis zu 8 C-Atomen, insbesondere $C_1$-$C_4$-Alkyl, ein geradkettiger oder verzweigter ungesättigter, aliphatischer Kohlenwasserstoffrest mit bis zu 4 C-Atomen, insbesondere $C_2$-$C_4$-Alkenyl, ein cycloaliphatischer Kohlenwasserstoffrest mit 5 - 7 C-Atomen, insbesondere $C_5$-$C_7$-Cycloalkyl, ein araliphatischer Kohlenwasserstoffrest mit 8 oder 9 C-Atomen, insbesondere Phenethyl oder Benzyl, oder ein Ammoniumion, das sich von einem primären, sekundären oder tertiären Amin ableitet, z.B. Wasserstoff, Methyl, Ethyl, n-Butyl, n-Pentyl, n-Hexyl, n-Heptyl, 2-Propyl, 2-Butyl, 2-Pentyl, 3-Hexyl, 2-Methylpropyl, 2-Methylbutyl, 4,4-Dimethylpentyl, 5,5-Dimethylhexyl, Cyclopentyl, Cyclohexyl, Cycloheptyl, Methylammonium, Dicyclohexylammonium, Tris-(hydroxymethyl)-methylammonium).

Unter den Substituenten $R^2$ sind die im folgenden aufgeführten besonders bevorzugt:

Unsubstituiertes Phenyl oder mit Halogen, Trifluormethyl, $C_{1-4}$-Alkyl oder $C_{1-4}$-Alkoxy einfach substituiertes Phenyl, geradkettiges oder verzweigtes $C_{3-7}$-Alkyl, das substituiert sein kann mit gegebenenfalls substituierten $C_{5-7}$-Cycloalkyl, mit $C_{1-3}$-Alkoxy, mit Phenoxy oder Halogenphenoxy, mit Thienyloxy oder Halogenthienyloxy, mit Cyclohexyloxy, mit Thienyl, mit Halogenthienyl oder mit Furyl, insbesondere die Reste:
n-Pentyl, 1,1-Dimethylpentyl, Cyclopentylmethyl, Cyclohexylmethyl, 1,1-Dimethyl-2-ethoxy-ethyl, 1,1-Dimethyl-2-methoxyethyl, 1,1-Dimethyl-cyclohexyloxymethyl, 1-Fluorpentyl, 1-Chlorpentyl, 5-Fluorpentyl, 5-Chlorpentyl, 3-Thienyl-2-ethyl, 2-Thienyl-2-ethyl, 3-(2-Chlorthienyl)-2-ethyl, 2-(5-Chlorthienyl)-2-ethyl, Phenoxymethyl, 3-Chlorphenoxymethyl, 2-Thienyloxymethyl, 3-(2-Chlorthienyl)-oxy-methyl, 2-(5-Chlorthienyl)-oxymethyl, 3-Furyl-2-ethyl, 2-Furyl-2-ethyl, 2,2,3,3-Tetrafluorcyclobutyl-2-ethyl, Phenyl, 3-Chlor-phenyl, 3-Trifluormethylphenyl.

n hat bevorzugt die Bedeutung 1 oder 2.

Gegenstand der Erfindung ist ferner ein Verfahren zur Herstellung der Verbindungen der Formel I, das dadurch gekennzeichnet ist, daß man

a) das Imidazolidinon der Formel II

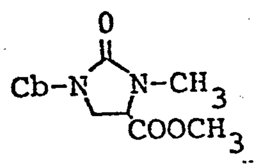

II

worin Cb den Rest

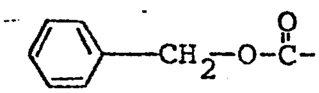

bedeutet

durch Reduktion der Esterfunktion in den Imidazolidinonalkohol III überführt,

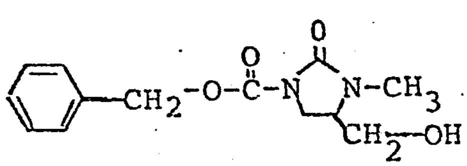

III

b) die Verbindung der Formel III oxydiert zu dem enolisierten Aldehyd der Formel IV

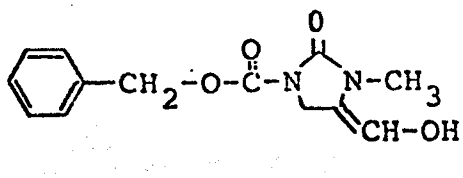 IV

c) den Aldehyd der Formel IV umsetzt mit einem Phosphonat der Formel V

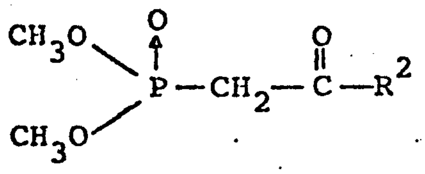 V

worin $R^2$ die zur Formel I gegebene Bedeutung hat, zu einem Enon der Formel VI

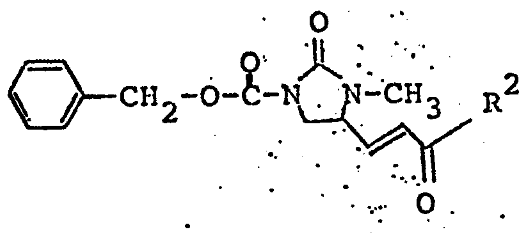 VI

worin $R^2$ die zur Formel I gegebene Bedeutung hat,

d) das Enon der Formel VI in bekannter Weise mit einem Reduktionsmittel zu einem 3'-Epimerengemisch der Alkohole der Formel VII reduziert, worin $R^2$ die zur Formel I gegebene Bedeutung hat, und gegebenenfalls das erhaltene 3'-Epimerengemisch der Alkohole der Formel VII nach üblichen Methoden in das α- und ß-Epimere auftrennt

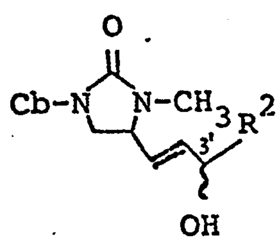 VII

e) in dem Epimerengemisch der Alkohole der Formel VII oder in den reinen α- bzw. ß-Epimeren die Cb-Schutzgruppe am N-Atom 3 durch alkalische Hydrolyse abspaltet, wobei man eine Verbindung der Formel VIII

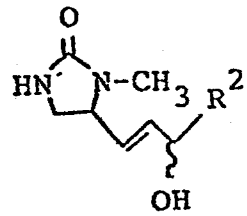

VIII

erhält, worin $R^2$ die zur Formel I gegebene Bedeutung hat, oder

e') das Epimerengemisch der Alkohole oder die reinen α- bzw. ß-Epimeren der Formel VII nach üblichen Methoden hydriert zu einer Verbindung der Formel IX

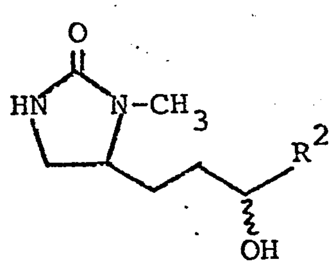

IX

worin $R^2$ die zur Formel I angegebene Bedeutung hat,

f) eine Verbindung der Formel VIII oder IX an der Alkoholfunktion acyliert zu einer Verbindung der Formel X

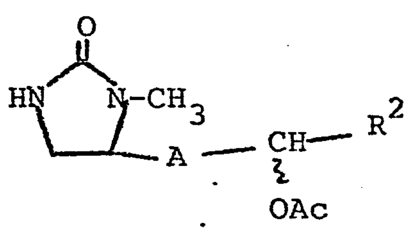

X

worin A eine -CH=CH- oder $-CH_2-CH_2-$Gruppe und Ac einen Alkanoyl-, Arylalkanoyl- oder Cycloalkanoylrest darstellt und $R^2$ die zur Formel I angegebene Bedeutung hat,

g) eine Verbindung der Formel X durch schwefelübertragende Reagenzien nach üblichen Methoden überführt in eine Verbindung der Formel XI

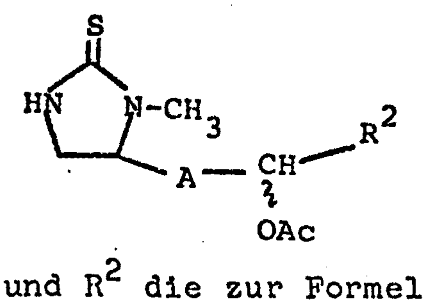

XI

worin A und $R^2$ die zur Formel I und Ac die zur Formel X angegebene Bedeutung haben,

h) in einer Verbindung der Formel XI durch alkalische Hydrolyse die Schutzgruppe Ac abspaltet, wobei man eine Verbindung der Formel XII

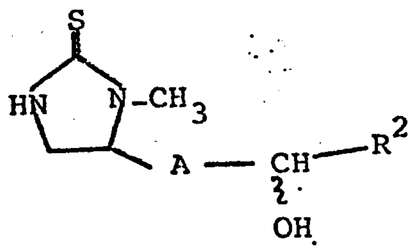

XII

erhält, worin A und $R^2$ die zur Formel I angegebene Bedeutung haben,

i) eine Verbindung der Formel XII mit einer Verbindung der Formel XIII

$$Hal-CH_2-CH_2-(CH_2)_n-COOR^1 \qquad XIII$$

worin $R^1$ und n die zur Formel I genannte Bedeutung haben und Hal Chlor, Brom oder Jod bedeutet, umsetzt zu einer Verbindung der Formel I,

k) gegebenenfalls eine Verbindung der Formel I, worin $R^1$ nicht gleich Wasserstoff oder ein Kation ist, verseift zu einer Verbindung der Formel I, worin $R^1$

Wasserstoff oder ein physiologisch verträgliches Kation bedeutet,

1) gegebenenfalls in einer Verbindung der Formel I, worin $R^1$ gleich Wasserstoff oder ein physiologisch verträgliches Metall-, $NH_4$ oder Ammoniumion bedeutet, das sich von einem primären, sekundären oder tertiären Amin ableitet, und $R^2$ und n die zur Formel I gegebenen Bedeutungen haben, das Kation $R^1$ gegen ein anderes austauscht.

Für die Herstellung des im erfindungsgemäßen Verfahren als Ausgangsmaterial verwendeten Imidazolidinons II kann man analog dem Verfahren arbeiten, das von S. Saijo und Mitarbeiter in Chem. Pharm. Bull 28, 1459 (1980) beschrieben wurde.

Den Imidazolidinalalkohol der Formel III erhält man, wenn man den Imidazolidinester der Formel II mit einem komplexen Metallhydrid, vorzugsweise einem Alkaliboranat wie Natrium-, Kalium- oder Lithium-borhydrid umsetzt.

Die Oxidation eines Alkohols der Formel III zu einem Aldehyd der Formel IV kann durch Oxidationsmittel wie Pyridiniumchlorochromat in inerten Lösungsmitteln wie Methylenchlorid oder Chloroform erfolgen. Eine weitere Möglichkeit der Oxidation besteht in der Reaktion mit Thioanisol/$Cl_2$/Trimethylamin in Tetrachlorkohlenstoff oder in der Umsetzung mit DMSO / Oxalylchlorid / $NEt_3$ bei -20°C. Der Aldehyd der Formel IV liegt vollständig in enolisierter Form vor.

Im weiteren wird Aldehyd der Formel IV nach Horner-Emmons-Wittig mit einem Phosphonsäureester der Formel V zu einem ungesättigten Keton der Formel VI umgesetzt, wobei eine bevorzugte Ausführungsform darin besteht, daß

man den Phosphonsäureester der Formel V in Dimethoxyethan mit DBU (1,8-Diazabicyclo-[5.4.0]-undec—7—en) versetzt und anschließend den Aldehyd der Formel IV zugibt und bei Raumtemperatur 2 - 6 Stunden reagieren läßt. Die Phosphonsäureester der Formel V können nach literaturbekannten Verfahren (siehe z.B. J. Am. Chem. Soc. 88, 5654 (1966)) hergestellt werden.

Die Verbindungen der Formel VII erhält man in Form ihrer Epimerengemische, wenn man ein Enon der Formel VI mit einem komplexen Metallhydrid, vorzugsweise mit Alkaliboranat oder mit D,L-Isobornyloxyaluminium-isopropoxid reduziert, vorzugsweise zwischen -5° und +20°C in Methanol, Ethanol oder Ethern wie DME, THF ggf. unter Wasserzusatz.

Die Verbindungen der Formel VIII erhält man durch hydrolytische Abspaltung der Schutzgruppe am N-3, vorzugsweise mit Alkalihydroxyden wie NaOH, KOH, LiOH in Alkohol-Wassergemischen bei +5° bis 30°C.

Die Acylierung der Verbindungen der Formel VIII oder IX erfolgt in der allgemein üblichen Weise mit Acylhalogeniden (wie z.B. Acetylchlorid, Benzylchlorid) etc.) oder mit Säureanhydriden (wie z.B. Essigsäureanhydrid, Propionsäureanhydrid etc.) in Gegenwart von Basen wie Pyridin, Triethylamin u.a..

Die Verbindungen der Formel XI lassen sich aus den Imidazolidionen der Formel X durch Umsetzung mit schwefelübertragenden Reagentien, wie z.B. Phosphorpentasulfid, Phosphorpentasulfid / Calciumoxid, Phosphorpentasulfid-Pyridin-Komplex oder Phosphorpentasulfid Anisol-Komplex in inerten Lösungsmitteln, wie z.B. Toluol, Dimethoxyethan oder Pyridin nach literaturbekannten Methoden herstellen (siehe z.B. Bull. Soc. Chim. Belg. 87, (3), 229 (1978).

In den Verbindungen der Formel XI läßt sich die Acylschutzgruppe in der allgemein üblichen Weise durch alkalische oder saure Hydrolyse abspalten, bevorzugt jedoch durch Rühren mit trockenen Kaliumcarbonat in Methanol bei Raumtemperatur.

Zur Darstellung der Verbindungen der Formel I werden die Imidazolidinthione der Formel XII mit den Alkylhalogeniden der Formel XIII alkyliert.

Diese Reaktion kann in einem inerten Lösungsmittel, z.B. Toluol, Diglyme, Tetrahydrofuran, Dimethoxyethan oder Dimethylformamid in Gegenwart einer Base, wie z.B. Pyridin, Triethylamin, Kaliumcarbonat oder Natriumhydrid bei 15 - 180°C durchgeführt werden. Eine bevorzugte Ausführungsform dieser Reaktion besteht jedoch darin, die Verbindungen der Formel XII in absolutem Diglyme ohne Basenzusatz mit den Verbindungen der Formel XIII bei 70 - 100°C innerhalb 1 - 6 Stunden zu alkylieren und aus dem intermediär entstehenden NH-Salzen der Verbindungen der Formel I bei der Aufarbeitung mit wäßriger $NaHCO_3$-Lösung die Verbindungen der Formel I freizusetzen.

Verbindungen der Formel I, worin $R^1$ nicht Wasserstoff oder ein Kation darstellt, können in alkalischem Medium zu Verbindungen der Formel I, worin $R^1$ Wasserstoff oder ein Kation bedeutet, verseift werden, z.B. mit NaOH oder KOH in einem niedermolekularen Alkohol wie Methanol oder in Ethern wie Dimethoxyethan oder THF, gegebenenfalls in Gegenwart von Wasser. Vorteilhafterweise stellt man die Verbindungen der Formel I, worin $R^1$ gleich Wasserstoff ist, jedoch dadurch her, daß man die Verbindungen der Formel XII mit einer Verbindung der Formel XIII, bei der $R^1$ gleich Wasserstoff ist, alkyliert. Verbindungen der Formel I, bei denen $R^1$ ein Kation darstellt, erhält man bevorzugt durch Umsetzung (Neutralisation) der Verbindungen der Formel I mit $R^1$ = Wasserstoff mit Metallhydroxyden wie z.B. NaOH, KOH, LiOH in wäßriger Lösung und Verdampfen des Lösungsmittels, vorzugsweise durch Gefriertrocknung.

Das Alkalikation läßt sich an Ionenaustauschern in üblicher Weise gegen beliebige Kationen austauschen. Dazu läßt man die Lösung des Alkalisalzes eines erfindungsgemäßen Imidazolinyl-thioethers der Formel I durch eine mit einem Kationenaustauscher, wie z.B. ®Amberlite CG-50 oder ®Dowex CCR-2 gefüllte Säule laufen. Der Kationenaustauscher ist mit dem erwünschten Kation beladen, z.B. mit einem Ammoniumion, das sich von einem primären, sekundären oder tertiären Amin ableitet. Das erwünschte Salz erhält man durch Eindampfen des Eluats.

Man kann Verbindungen der Formel I, bei denen $R^1$ = $NH_4$ oder ein Ammoniumion bedeutet, das sich von einem primären, sekundären oder tertiären Amin ableitet, auch herstellen, indem man Verbindungen der Formel I, bei denen $R^1$ = Wasserstoff bedeutet, in einer alkoholischen Lösung mit einer äquimolaren Menge des entsprechenden Amins versetzt und das Lösungsmittel eindampft.

Verbindungen der Formel I, worin $R^1$ Wasserstoff oder ein Kation bedeutet, lassen sich zu Verbindungen der Formel I verestern, worin $R^1$ die übrigen zur Formel I gegebenen Bedeutungen hat. So kann man z.B. Verbindungen der Formel I mit $R^1$ = H bei Temperaturen zwischen -40° und +20°C mit einem Diazoalkan verestern, wobei die üblichen Lösungsmittel wie z.B. Diethylether, Tetrahydrofuran, Chloroform oder niedermolekulare Alkohole wie Methanol verwendet werden können. Die resultierenden Ester können in einfacher Weise durch Eindampfen des Lösungsmittels isoliert und ggf. chromatografisch gereinigt werden. Eine Veresterungsmethode besteht darin, daß man Salze

der Verbindungen der Formel I ($R^1$ = Kation) in Gegenwart einer Base wie z.B. eines Metallalkoholates oder Metallcarbonates in einem geeigneten Lösungsmittel mit einem Alkylierungsmittel $R^1$-Z umsetzt. Als Metallalkoholate kommen z.B. Natriummethylat, Natriumethylat oder Kaliumtertiärbutylat in Betracht, als Carbonat eignet sich z.B. Kaliumcarbonat. Als geeignetes Lösungsmittel kommen Alkohole wie z.B. Methanol oder tert. Butanol, Ether wie Tetrahydrofuran oder 1,2-Dimethoxyethan und insbesondere dipolare aprotische Lösungsmittel wie Dimethylformamid, Dimethylsulfoxyd, Acetonitril, oder N-Methylpyrrolidon in Betracht. In der Formel $R^1$-Z bedeutet Z vorzugsweise Brom oder Jod oder einen Sulfonsäurerest. Zur Darstellung von Estern der Formel I ($R^1$ = Alkyl) eigent sich auch die Methode der Umesterung mit Überschuß an Alkoholen wie z.B. Methanol, Ethanol, Isopropanol.

Die Verbindungen der Formel I fallen als Racemat bezüglich der Stellung am Kohlenstoffatom 5 des Imidazolinrings sowie als α/ß-Isomere bezüglich des Kohlenstoffatoms 3' an. Die Trennung der α/ß-Isomeren erfolgt bevorzugt auf der Stufe der Endprodukte der Formel I. Die Trennung des Racemats bezüglich der Kohlenstoffatome 5 des Imidazolinrings kann bevorzugt bei den Verbindungen der Formel VII, VIII oder IX erfolgen, oder auf der Stufe der Endprodukte der Formel I. Das bedeutet, daß alle beschriebenen Reaktionen mit Epimerengemischen, reinen Epimeren oder optisch aktiven Antipoden durchgeführt werden können. Die beanspruchten Verbindungen der Formel I umfassen daher Diastereomerengemische, reine Diastereomere, Epimerengemische und reine Epimere.

Sofern die einzelnen Reaktionsprodukte nicht bereits in genügend reiner Form anfallen, so daß sie für den folgenden Reaktionsschritt eingesetzt werden können, empfiehlt sich eine Reinigung mittels z.B. Säulen-, Dünnschicht- oder Hochdruckflüssigkeitschromatographie.

Außer den in den Beispielen beschriebenen Verbindungen lassen sich nach den erfindungsgemäßen Verfahren die folgenden Verbindungen herstellen:

1-Methyl-2(1-Thia-5-carboxy-pentyl)-5(3-hydroxy-1-octenyl)-Δ2-imidazolin
1-Methyl-2(1-Thia-4-carboxy-butyl)-5(3-hydroxy-1-octenyl)-Δ2-imidazolin
1-Methyl-2(1-Thia-6-carboxy-hexyl)-5(3-hydroxyl-1-octenyl)-Δ2-imidazolin
1-Methyl-2(1-Thia-4-methoxycarbonyl-butyl)-5(3-hydroxy-1-octenyl)-Δ2-imidazolin
1-Methyl-2(1-Thia-5-methoxycarbonyl-pentyl)-5(3-hydroxy-1-octenyl)-Δ2-imidazolin
1-Methyl-2(1-Thia-4-methoxycarbonyl-butyl)-5(3-hydroxy-5(2-furyl)-1-pentenyl)-Δ2-imidazolin
1-Methyl-2(1-Thia-4-methoxycarbonyl-butyl)-5(3-hydroxy-4,4-dimethyl-4-cyclohexyloxy-1-butenyl)-Δ2-imidazolin
1-Methyl-2(1-Thia-4-methoxycarbonyl-butyl)-5(3-hydroxy-3-phenyl-1-propenyl)-Δ2-imidazolin
1-Methyl-2(1-Thia-4-ethoxycarbonyl-butyl)-5(3-hydroxy-4(3-trifluormethyl-phenyloxy)1-butenyl-Δ2-imidazolin
1-Methyl-2(1-Thia-4-cyclohexyloxycarbonyl-butyl)-5(3-hydroxy-1-octenyl)-Δ2-imidazolin
1-Methyl-2(1-Thia-7-methoxycarbonyl-heptyl)-5(3-hydroxy-1-octenyl)-Δ2-imidazolin
1-Methyl-2(1-Thia-4-ethoxycarbonyl-butyl)-5(3-hydroxy-1-nonenyl)-Δ2-imidazolin
1-Methyl-2(1-Thia-4-methoxycarbonyl-butyl)-5(3-hydroxy-1-decenyl)-Δ2-imidazolin
1-Methyl-2(1-Thia-5-carboxy-pentyl)-5(3-hydroxy-1-octanyl)-Δ2-imidazolin
1-Methyl-2(1-Thia-4-carboxy-butyl)-5(3-hydroxy-1-octanyl)-Δ2-imidazolin

1-Methyl-2(1-Thia-6-carboxy-hexyl)-5(3-hydroxy-1-octanyl) -Δ2-imidazolin

1-Methyl-2(1-Thia-4-methoxycarbonyl-butyl)-5(3-hydroxy-1-octanyl)-Δ2-imidazolin

1-Methyl-2(1-Thia-5-methoxycarbonyl-pentyl)-5(3-hydroxy-1-octanyl)-Δ2-imidazolin

1-Methyl-2(1-Thia-4-methoxycarbonyl-butyl)-5(3-hydroxy-5(2-furyl)-1-pentanyl)-Δ2-imidazolin

1-Methyl-2(1-Thia-4-methoxycarbonyl-butyl)-5(3-hydroxy-4,4-dimethyl-4-cyclohexyloxy-1-butanyl)-Δ2-imidazolin

1-Methyl-2(1-Thia-4-methoxycarbonyl-butyl)-5(3-hydroxy-3-phenyl-1-propanyl)-Δ2-imidazolin

1-Methyl-2(1-Thia-4-ethoxycarbonyl-butyl)-5(3-hydroxy-4(3-trifluormethyl-phenyloxy)1-butanyl)-Δ2-imidazolin

1-Methyl-2(1-Thia-4-cyclohexyloxycarbonyl-butyl)-5(3-hydroxy-1-octanyl)-Δ2-imidazolin

1-Methyl-2(1-Thia-7-methoxycarbonyl-heptyl)-5(3-hydroxy-1-octanyl-Δ2-imidazolin

1-Methyl-2(1-Thia-4-ethoxycarbonyl-butyl)-5(3-hydroxy-1-nonayl)-Δ2-imidazolin

1-Methyl-2(1-Thia-4-methoxycarbonyl-butyl)-5(3-hydroxy-1-decanyl)-Δ2-imidazolin

Die Verbindungen der Formel I zeichnen sich aus durch hemmende Wirkung auf die Thrombocytenaggregation, Relaxation der Gefäßwand, sowie blutdrucksenkende Eigenschaften. Sie können daher als Arzneimittel angewandt werden. Die Anwendung der Verbindungen der Formel I als Blutdrucksenker erfolgt im Tagesdosisbereich von 0,01 mg/kg - 0,5 mg/kg, vorzugsweise 0,05 mg/kg - 0,1 mg/kg bei i.v. Applikation bzw. Tagesdosisbereich von 0,05 mg/kg - 2 mg/kg, vorzugsweise 0,1 - 1 mg/kg bei oraler Applikation. Zur Relaxation der Gefäßwand, besonders der Coronararterien sowie zur Hemmung der Thrombocytenaggregation kommen die gleichen Tagesdosen, teilweise auch niedere Dosierungen , wie oben angegeben, in Betracht.

Die Verbindungen sind auch brauchbar bei Säugetieren einschließlich Menschen sowie bestimmten Nutztieren, z.B. Hunden und Schweinen, zur Verminderung und Steuerung übermäßiger Magensaftsekretion, womit die Bildung von Magen-Darmgeschwüren vermindert oder vermieden werden und die Heilung solcher bereits vorhandener Geschwüre beschleunigt werden kann. Zu diesem Zweck werden die Verbindungen neben der oralen Anwendung intravenös, subkutan oder intramuskulär injiziert oder infundiert. Das Dosierungsschema für das Prostacyclin hängt bei dieser Behandlung von verschiedenen Faktoren ab einschließlich Typ, Alter, Gewicht, Geschlecht und medizinischem Zustand des Patienten, dem Dosierungsschema des entzündungshemmenden Synthetase-Inhibitor bezüglich der Magen/Darmwirkung. So empfindet z.B. nicht jeder Patient, der eine entzündungshemmende Substanz benötigt, die gleichen unangenehmen gastrointestinalen Effekte. Diese ändern sich vielmehr in der Art und Ausmaß. Es liegt daher im Erfahrungsbereich des Arztes oder Tierarztes festzustellen, ob die Verabreichung der entzündungshemmenden Substanz unerwünschte gastrointestinale Effekte beim Menschen oder Tier erzeugt. und die wirksame Menge des Prostaglandins zu verschreiben, mit der diese Effekte im wesentlichen eliminiert werden können. Einzelne Vertreter dieser Substanzen eignen sich zur Behandlung von Asthma. Sie sind beispielsweise nützlich als Bronchodilatoren oder als Inhibitoren von Mediatoren wie z.B. SRS-A und Histamin, die aus durch einen Antigen / Antikörper-Komplex aktivierten Zellen freigesetzt werden. Die Verbindungen bekämpfen daher Krämpfe und erleichtern das Atmen bei Krankheitszuständen wie Bronchitis, Pneumonie und Emphysem. Für diese Zwecke werden die Verbindungen in verschiedenen Dosierungsformen verabreicht, z.B. oral in Form von Tabletten, Kapseln oder Flüssigkeiten, rektal in Form von Suppositorien, parenteral, subkutan oder intramuskulär, wobei intravenöse Verabreichung in Notsituationen bevorzugt wird.

Die erfindungsgemäßen Verbindungen der Formel I können als freie Säuren, oder in Form ihrer physiologisch unbedenklichen anorganischen oder organischen Salze oder als Ester zur Anwendung kommen. Säuren und Salze bzw. Ester können in Form ihrer wäßrigen Lösungen oder Suspensionen oder auch gelöst oder suspendiert in pharmakologisch unbedenklichen organischen Lösungsmitteln wie ein- oder mehrwertigen Alkoholen wie z.B. Ethanol, Ethylenglykol oder Glycerin, in Triacetin, in Alkohol-Acetalaldehyddiacetalgemischen, Ölen wie z.B. Sonnenblumenöl oder Lebertran, Ethern wie z.B. Diethylenglykoldimethylether oder auch Polyethern wie z.B. Polyethylenglykol oder auch in Gegenwart anderer pharmakologisch unbedenklicher Polymerträger wie z.B. Polyvinylpyrrolidon zur Anwendung gelangen.

Als Zubereitungen können die üblichen galenischen Infusions- oder Injektionslösungen und Tabletten sowie örtlich anwendbare Zubereitungen wie Cremes, Emulsionen, Suppositorien oder Aerosole in Frage kommen.

Die Verbindungen der Formel III - XIII sind neue wertvolle Zwischenprodukte für die Herstellung von Verbindungen der Formel I.

Beispiel 1

1-Methyl-2-oxo-3-benzyloxycarbonyl-5-hydroxymethyl-imidazolidin III

25 g (86 mMol) 1-Methyl-2-oxo-3-benzyloxycarbonyl-5-carbomethoxy-imidazolidin (hergestellt wie von S. Saijo et al. in Chem. Pharm. Bull. 28, 1459 (1980) beschrieben) werden in 200 ml Ethanol abs. gelöst und auf 0°C unter Rühren abgekühlt. In Portionen gibt man 3,8 g (98 mMol) $NaBH_4$ zu. Nach beendeter Zugabe wird ca. 1,5 Stunden bei 0°C gerührt. D.C. in $CH_2Cl_2$MEOH 10 : 1. Nach beendeter Reaktion wird mit 2nHCl angesäuert bis pH = 3 und das Reaktionsgemisch im Vakuum eingeengt. Der Rückstand wird in $CH_2Cl_2$ aufgenommen, mit wenig $H_2O$ versetzt. Phasen trennen, $H_2O$-Phase noch 3 x mit $CH_2Cl_2$ eluieren. Vereinigte $CH_2$-$Cl_2$-Phasen trocknen, mit $MgSO_4$ filtrieren und einengen im Vakuum.

Ausbeute:
21 g n.H.V., Fp.: 73 - 79°C, helle Kristalle, (92 % d. Th.) $C_{13}H_{16}O_4N_2$ MG = 264

NMR:
($CDCl_3$) δ ppm 270 MHz
2,8 (s,3H)N-$CH_3$; 3,0-3,3 (breites s, 1H) OH; 3,5-3,9 (m,5H) N-$CH_2$-, N-CH , -$CH_2$O-; 5,2(s,2H) $C_6H_5CH_2O$-; 7,25-7,45 (m, 5H) $C_6H_5$;

Rf-Wert:
Methylenchlorid/Methanol 10:1 = 0,39

Beispiel 2

1-Methyl-2-oxo-3-benzyloxycarbonyl-5-formyl-imidazolidin IV

0,7 ml DMSO abs. (9,6 mMol) in 10 ml $CH_2Cl_2$ abs. werden unter Feuchtigkeitsausschluß auf -60°C abgekühlt. Bei dieser Temperatur werden 0,4 ml (4,4 mMol) Oxalylchlorid zugegeben und ca. 10 Minuten gerührt. Anschließend wird 1,05 g (4 mMol) 1-Methyl-2-oxo-3-benzyloxycarbonyl-5-hydroxymethyl-imidazolidin (Beispiel 1) gelöst in 10 ml $CH_2Cl_2$ abs. zugetropft. Es wird 20 Minuten bei -60°C weiter gerührt. Dann wird 2,7 ml TÄA zugesetzt und 30 Minuten gerührt. Bei -10°C bis -20°C wird dann mit ethanolischer HCl auf pH = 5 gestellt. D.C. $CH_2Cl_2$ MEOH 10 : 1. Das Reaktionsgemisch wird am Rotavapor eingeengt. Der Rückstand wird auf Kieselgel chromatographiert. Elutionsmittel $CH_2Cl_2$/MEOH 10 : 1.

Ausbeute:

960 mg, helles Öl
(92 % d. Th.) $C_{13}H_{14}O_4N_2$ MG 262

NMR:

($CDCl_3$)δ ppm 60 MHz
2,8 (s,3H)N-$CH_3$; 3,1-4,0 (m,3H) N-$CH_2$, OH;
4,3-4,8 (m,1H) =CH OH; 5,15 (s,2H) $C_6H_5CH_2O$-;
7,2 (s,5H) $C_6H_5$.

Rf-Wert:

Methylenchlorid/Methanol 10 : 1 = 0,42

Beispiel 3 a

1-Methyl-2-oxo-3-benzyloxycarbonyl-5-(3-oxo-octenyl)-imidazolidin VI

9,6 g (63 mMol) 1,8-Diazabicyclo-(5,4,0)-undec-7-en (DBU) werden in 60 ml Dimethoxyethan (DME) abs. vorgelegt. Dazu tropft man bei Raumtemperatur 13,9 g (63 mMol) 2-Oxoheptyl-phosphonsäuredimethylester gelöst in 150 ml DME abs. Es wird 30 Minuten bei Raumtemperatur gerührt. Anschließend werden 15 g (33 mMol) 1-Methyl-2-oxo-3-benzyloxycarbonyl-5-formyl-imidazolidin (Beispiel 2) gelöst in 150 ml DME abs. zugetropft. Nach beendeter Zugabe wird ca. 25 Minuten bei Raumtemperatur gerührt. Nach beendeter Reaktion wird mit 2nHCl vorsichtig unter Eiskühlung auf pH 7 gestellt. Das Reaktionsgemisch wird eingeengt, der Rückstand in EE aufgenommen und mit NaCl-Lösung gewaschen. EE-Phase trocknen und im Vakuum einengen. Rohausbeute: 20 g.
Säulenchromatographie auf $SiO_2$ mit EE als Elutionsmittel,

Ausbeute:

9,0 g, farblose Kristalle, Fp. = 50 - 53°C
(63 % d. Th.) $C_{20}H_{26}N_2O_4$ MG = 358

NMR:

($CDCl_3$)δ ppm 270 MHz
0,8-0,95 (t,3H) $CH_3$; 1,2-1,4 (m,4)-$CH_2$-; 1,5-1,7 (m,2H) $CH_2$; 2,55 (t,2H) $CH_2CO$; 2,8 (s,3H) N-$CH_3$; 3,6 (m,1H) >CH-N; 4,0-4,15 (m,2H)N-$CH_2$; 5,3 (s,2H) $C_6H_5CH_2$; 6,2-6,6 (ABX-Spektrum,2H) CH=CH; 7,3-7,5 (m,5H) $C_6H_5$.

Rf-Wert:

Essigester 0,66 (mit Jod anfärbbar)

Beispiel 3 b-3 i

In Analogie zu Beispiel 3 a lassen sich aus den entsprechenden Phosphonaten der allg. Formel V und 1-Methyl-2-oxo-3 - benzyloxycarbonyl-5-formyl-imidazolidin IV (n=1) die Verbindungen 3 b - 3 i darstellen.

| Beispiel Nr. | Rf-Werte Essigester | $R^2$ = | Ausbeute % |
|---|---|---|---|
| b) | 0.72 | 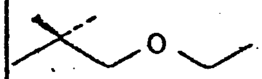 | 69 |
| c) | 0.8 | 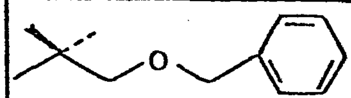 | 76 |
| d) | 0.79 | 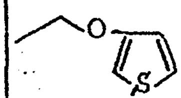 | 61 |
| e) | 0.68 | 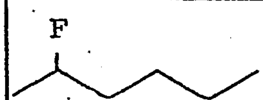 | 48 |
| f) | 0.64 | 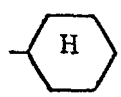 | 81 |
| g) | 0.7 | 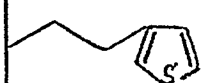 | 60 |
| h) | 0.83 | 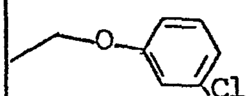 | 52 |
| i) | 0.68 | 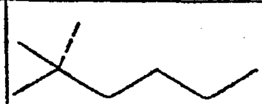 | 45 |

Beispiel 4

1-Methyl-2-oxo-3-benzyloxycarbonyl-5-(3-hydroxy-octenyl) imidazolidin VII

8,5 g 1-Methyl-2-oxo-3-benzyloxycarbonyl-5-(3-oxo-octenyl)-imidazolidin (Beispiel 3) werden in 250 ml Methanol und 140 ml Wasser gelöst. Bei 0° werden unter Rühren 3,8 g Natriumborhydrid, in 100 ml Eiswasser gelöst, zugegeben. Nach 2 - 3 Stunden ist die Reaktion beendet. Das Reaktionsgemisch wird mit 2N Salzsäure auf pH 4 angesäuert und anschließend im Vakuum eingeengt. Der Rückstand wird in Essigester aufgenommen, mit Kochsalzlösung gewaschen, die Wasser-Phase 3x mit Essigester extrahiert. Die vereinigten organischen Extrakte werden mit $MgSO_4$ getrocknet, filtriert und eingeengt.

Ausbeute:

7,9 g, helles öl (95 % d. Th.) $C_{20}H_{28}N_2O_4$ MG = 360

NMR: δ-Werte in ppm (60 MHz)

0,8-1,1 (m,3H) $CH_3$, 1,1-1,8 (m,8H) $CH_2$, 2,85 (s,3H)N-$CH_3$,3,4-4,3 (m, 4H) N-$CH_2$, N-CH, CH-OH; 5,25 (s,2H)$CO_2CH_2C_6H_5$; 5,5-6,05 (m, 2H) CH=CH; 7,2-7,5 (m,5H) $C_6H_5$.

Rf-Wert:

Essigester 3'ß-Epimeres : 0,54
3'α-Epimeres : 0,44

Dünnschichtkieselgel-Platten Merck: Entwickeln mit Jod.

0142027

Beispiel 5

1-Methyl-2-oxo-5-(3-hydroxy-octenyl)-imidazolidin VIII

8 g 1-Methyl-2-oxo-3-benzyloxycarbonyl-5-(3-hydroxy-octenyl)-imidazolidin (Beispiel 4) in 250 ml Methanol werden bei Raumtemperatur unter Rühren mit 110 ml 2N NaOH versetzt und 4 Stunden weitergerührt. Das Reaktionsgemisch wird im Vakuum eingeengt und der Rückstand mit Methylenchlorid mehrmals extrahiert. Die Methylenchloridphase wird mit Kochsalzlösung gewaschen, getrocknet mit $MgSO_4$, filtriert und im Vakuum eingeengt - ergibt 9,0 g Öl. Säulenchromatographie mit Kieselgel 60 (Merck AG) 0,063 - 0,2 mm, Elutionsmittel Essigester.

Ausbeute:

5,0 g Kristalle Fp. 82 - 85°C
(98 % d.Th.) $C_{12}H_{22}O_2N_2$ MG = 226

NMR:

($CDCl_3$)δ ppm
0,9 (t, 3H) $CH_3$; 1,0 - 1,5 (m, 6H) $CH_2$; 1,5 - 1,9 (m,2H) $CH_2$; 2,7 (s,3H) $NCH_3$; 3,0 - 4,3 (m,4H) $NCH_2$, HCN, CHOH; 4,6 (breites s, 1H) NHCO; 5,5 - 5,8 (m,2H) CH=CH;

Rf-Wert:

Essigester: 3ß'-Epimeres: 0,38
3α'-Epimeres: 0,26

Beispiel 6

1-Methyl-2-oxo-5-(3-acetoxy-octenyl)-imidazolidin X

5 g 1-Methyl-2-oxo-5-(3-hydroxy-otenyl)-imidazolidin (Beispiel 5) werden in 60 ml Pyridin gelöst und unter Rühren und Feuchtigkeitsausschluß bei Raumtemperatur innerhalb 8 Stunden portionsweise mit insgesamt 10 ml Acetanhydrid versetzt. Das Reaktionsgemisch wird auf Eiswasser gegeben und mit Essigester überschichtet. Unter Rühren wird mit 2N HCl bis pH = 3 angesäuert. Die organische Phase wird abgetrennt und die wäßrige Phase wird mehrmals mit Essigester extrahiert. Die vereinigten organischen Extrakte werden mit $MgSO_4$ getrocknet, filtriert und das Lösungsmittel im Vakuum entfernt.

Ausbeute:

5,6 g helle Kristalle Fp. 60 - 63°C
(94,5 % d.Th.) $C_{14}H_{24}N_2O_3$ MG = 268

NMR:

$(CDCl_3)$ δ ppm.
0,9 (+,3H) $CH_3$; 1,0-1,8 (m,8H) $CH_2$; 2,05 (s, 3H) OAc;
2,7 (s,3H)N-$CH_3$; 2,9 - 4,0 (m,3H) $NCH_2$, NCH;
4,5 (breites s, 1H) NHCO; 5,1 - 5,4 (m,1H) CH-OAc;
5,5 - 5,8 (m,2H) CH=CH;

Rf-Wert:

Essigester: 0,46

Beispiel 7

1-Methyl-2-thioxo-5-(3-acetoxy-octenyl)-imidazolidin XI

500 mg 1-Methyl-2-oxo-5-(3-acetoxy-octenyl)-imidazolidin (Beispiel 6) werden in 15 ml abs. Toluol gelöst. Nach Zugabe von 3 g Seesand wird im Stickstoffstrom auf 90°C erwärmt. Bei dieser Temperatur werden portionsweise 3 x 250 mg $P_4S_{10}$·Anisol zugegeben und 2 - 3 Stunden gerührt. Das Reaktionsgemisch wird filtiert, der Filterrückstand mit Toluol gewaschen und die Toluolphase im Vakuum eingeengt. Der Rückstand wird auf einer Merck-Fertigsäule mit Kieselgel 0,063 - 0,2 mm und Cyclohexan/Essigester = 1 : 1 als Elutionsmittel chromatographiert.

Ausbeute:

400 mg Fp.: 88 - 91°C
(76 % d. Th.) $C_{14}H_{24}SN_2O_2$ MG = 284

NMR: δ-Werte in ppm 60 MHz ($CDCl_3$)

δ = 0,8 - 1,9 (m,11H) $CH_2$, $CH_3$; 2,65 (s, 3H) OAc; 3,0 (s,3H) N-$CH_2$; 3,1 - 3,9 (m,2H)) N-$CH_2$; 4,0 - 4,4 (m, 1H) H-CH-C=; 4,4 (breites s, 1H) CSNH; 5,50 - 5,4 (m,1H) CH-OAc; 5,5 - 5,7 (m,2H) CH=CH;

Rf-Wert:

Essigester: 0,84

Beispiel 8

1-Methyl-2-thioxo-5-(3-hydroxy-octenyl)-imidazolidin XII

340 mg 1-Methyl-2-thioxo-5-(3-acetoxy-octenyl)-imidazolidin (Beispiel 7) werden in 20 ml abs. Methanol gelöst. Unter Rühren werden 400 g fein gepulvertes wasserfreies $K_2CO_3$ zugegeben. Es wird mehrere Stunden unter Feuchtigkeitsausschluß gerührt. Dann wird das Reaktionsgemisch mit 2N Essigsäure auf pH 6 gestellt und im Vakuum eingeengt. Der Rückstand wird in Essigester aufgenommen und die Essigesterphase mit Wasser extrahiert, mit $MgSO_4$ getrocknet, filtriert und im Vakuum eingeengt.

Ausbeute:

254 mg
(88 % d. Th.) $C_{12}H_{22}SN_2O$ MG = 242

NMR: δ-Werte in ppm 60 MHz (CDCl$_3$)

δ = 0,8 - 1,6 (m,11H) $CH_2$, $CH_3$; 3,0 (s,3H) N-$CH_3$; 3,1 - 4,4 (m,4H) N-$CH_2$, -N-CH, CH~~~OH; 5,6 - 5,8 (m,2H) olefinische Protonen; 5,9 (breites s, 1H) CSNH;

Rf-Wert:

Essigester: 3'ß-Epimeres 0.66
3'α-Epimeres 0.56

Beispiel 9 a

1-Methyl-2-(1-thia-4-ethoxycarbonyl-butyl)-5-(3-hydroxy-octenyl)-Δ2-imidazolin I

(A = CH=CH, $R^1$ = $C_2H_5$, $R^2$ = $C_5H_{11}$; n = 1)

175 mg 1-Methyl-2-thioxo-4-(3-acetoxy-octenyl)-imidazolin (Beispiel 8) werden in 5 ml abs. Diglyme gelöst und unter Rühren und Feuchtigkeitsausschluß auf 90°C erwärmt. Nach Zugabe von 0,2 ml 4-Brombuttersäureethylester wird 2 Stunden bei 90°C gerührt und dann erneut 0,2 ml 4-Brombuttersäureethylester zugegeben. Nach 4 - 5 Stunden bei 90°C ist die Reaktion beendet. Das Reaktionsgemisch wird mit Essigester versetzt und mit halbgesättigter $NaHCO_3$-Lösung gewaschen. Die organische Phase wird abgetrennt, mit $MgSO_4$ getrocknet, filtriert und eingeengt. An Kieselgel wird mit $CH_2Cl_2$ : MeOH 20 : 1 chromatographiert.

Ausbeute: 171 mg (66 % d.Th.) m.p. 43 - 46°C

Diastereomerengemisch: Rf-Wert = 0.22 ($CH_2Cl_2$: MeOH=10:1) (Diastereomerentrennung durch HPLC möglich) (Waters, µ-Bondapak-Alkyl-Phenyl 10 µm. 0,005 M $KH_2PO_4$ / $CH_3CN$ 4 : 1 (v/v), pH 3,0 ($H_3PO_4$))

$C_{18}H_{32}N_2SO_3$ MG 356

NMR ($CDCl_3$)
δ-Werte in ppm-Spektren für α- und ß-Epimeres im Rahmen der üblichen Auflösung identisch:

0,87 (t 3H) $CH_3$; 1,25 (t,3H) $OCH_2CH_3$; 1,23-1,45 (m,6H) $CH_2$; 1,46-1,62 (m,2H) $CH_2$; 2,04 (p,2H) $SCH_2$ $CH_2$ $CH_2$ $CO_2Et$; 2,45 (t,2H) $CH_2CO_2Et$; 2,66 (s,3H) N-$CH_3$; 3,14 (t,2H) $SCH_2$; 3,37-3,47 (m,1H) $NCH_2$; 3,83-4.00 (m,2H) >$NCH_2$, N-CH; 4,12 (q,2H)$OCH_2$; 4,1-4,2 (m,1H) CHOH; 5,56-5,77 (m,2H) CH=CH.

| Beispiel Nr. 9 | NMR-Daten (ppm) charakteristische Signale | $R^2$= | MS (Molmasse) | Rf-werte $CH_2Cl_2$/MeOH 10 : 1 α/β-Epimer | Ausbeute % |
|---|---|---|---|---|---|
| b | $\delta$ = 0.9 (s,6H) $-C(CH_3)_2$; 1.15 (t,3H) $-OCH_2CH_3$; 3.3 (s,2H) $-OCH_2$; 3.5 (q,2H) $OCH_2CH_3$; | 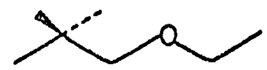 | $C_{19}H_{34}N_2SO_4$ 386.56 | 0.27 | 61 % |
| c | $\delta$ = 0.9 (d,6H) $C(CH_3)_2$; 7.2 (s,5H) aromat. Prot. | 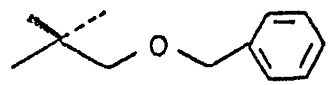 | $C_{24}H_{36}N_2SO_4$ 448.63 | 0.32 | 72 % |
| d | $\delta$ = 3.9 (d,2H) $CH_2O$, 6.1 - 7.3 (dreifacher m,3H) Thiopen | 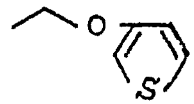 | $C_{18}H_{26}N_2S_2O_4$ 398.55 | 0.30 | 52 % |
| e | $\delta$ = 4.95 (m,1H) > CH-F | 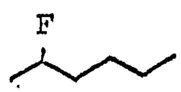 | $C_{18}H_{31}N_2SO_3F$ 374.52 | 0.24 | 56 % |
| f | $\delta$ = 1.1-2.0 (m,11H) CH, $-CH_2$; | 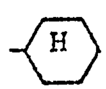 | $C_{18}H_{32}N_2SO_3$ 356.53 | 0.25 | 74 % |

0142027

| Beispiel Nr. 9 | NMR-Daten (ppm) charakteristische Signale | $R^2$ = | MS (Molmasse) | Rf-werte $CH_2Cl_2$/MeOH 10 : 1 α/β-Epimer | Ausbeute % |
|---|---|---|---|---|---|
| g | δ = 6.8-7.3 (m,3H) Thiophen; | 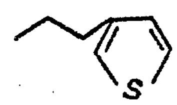 | $C_{19}H_{28}N_2S_2O_3$ 396.58 | 0.31 | 57 % |
| h | δ = 3.85 (d,2H) $-CH_2-O-$ 6.0-7.3 (m,4H) aromat. Protonen | 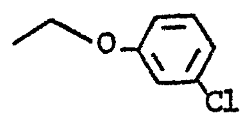 | $C_{20}H_{27}N_2SO_4Cl$ 426.96 | 0.34 | 48 % |
| i | δ = 0.85 (s,6H) $-C(CH_3)_2-$ | 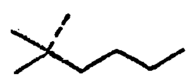 | $C_{20}H_{36}N_2SO_3$ 384.59 | 0.28 | 62 % |

Beispiel 10

1-Methyl-2-oxo-3-benzyloxy-carbonyl-5-(3-hydroxyoctanyl)-imidazolidin IX

20.7 g (57 mMol) 1-Methyl-2-oxo-3-benzoyloxymethyl-5-(3-hydroxyoctenyl)-imidazolidin (Beispiele 4) in 0,50 Liter Methanol werden mit 6,3 g Pd/C (10 %ig) versetzt und in der Schüttelente bei Normaldruck mit Wasserstoff hydriert. Nach Aufnahme der berechneten Menge Wasserstoff wird vom Katalysator abfiltriert und das Filtrat eingeengt.

Ausbeute:

13 g helles Öl
(100 % d. Th.) $C_{12}H_{24}N_2O_2$ MG = 228

NMR:
($CDCl_3$) δppm 60 MHz
0,9-1,8 (m,15H) $CH_3$, $CH_2$; 2,75 (s,3H) N-$CH_3$; 2,9-3,8 (m,3H) N-$CH_2$, N-CH ; 3,9-4,2 (m,1H) CH-OH; 5,6-5,9 (breites s, NHCO).

Rf-Wert:
$CH_2Cl_2/CH_3OH$ 20 : 1 = 0,44

Beispiel 11

1-Methyl-2-oxo-5-(3-acetoxy-octanyl)-imidazolidin X

13 g (57 mMol) 1-Methyl-2-oxo-5-(3-hydroxy-octanyl)-imidazolidin (Beispiel 10) werden in 100 ml abs. Pyridin gelöst. Dann gibt man portionsweise bei Raumtemperatur 6 ml (62 mMol) Acetanhydrid (frisch destilliert) zu und rührt 2 Tage bei Raumtemperatur. Im Vakuum wird das

Pyridin entfernt und der Rückstand in Essigester gelöst und 3 x mit Kochsalzlösung extrahiert. Die organische Phase wird getrocknet mit $MgSO_4$, im Vakuum eingeengt. Der Rückstand wird über Säulenchromatographie an Kieselgel mit $CH_2Cl_2/MeOH = 20 : 1$ als Elutionsmittel chromatographiert.

Ausbeute:
12,5 g hellgelbes Öl
(81 % d. Th.) $C_{14}H_{26}N_2O_3$ MG = 270

NMR:
($CDCl_3$) δppm 270 MHz
0,85 - 0,95 (t,3H) $CH_3$; 1,2 - 1,4 (m,6H) $CH_2$; 1,45 - 1,6 (m,4H) $CH_2$; 1,7 - 1,8 (m,2H) $CH_2$; 2,05 (s,3H) $COCH_3$; 2,74 (s,3H) N-$CH_3$; 3,05 u. 3,5 (t,2H) N-$CH_2$; 3,45 - 3,55 (m,1H)-N-CH< ; 4,5-4,55 (breites s,1H) CONH; 4,8 - 4,95 (m,1H)-CH-OAc;

Rf-Wert:
Methylenchlorid/Methanol 20:1 = 0,37

Beispiel 12

1-Methyl-2-thioxo-5-(3-acetoxy-octanyl)-imidazolidin XI

2,5 g (9,2 mMol) 1-Methyl-2-oxo-5-(3-acetoxy-octanyl)-imidazolidin (Beispiel 11) werden in 80 ml Toluol abs. gelöst, dazu gibt man 10 g Seesand und erwärmt unter Rühren auf 90°C. Anschließend werden 2,50 g $P_4S_{10}$·Anisol zugegeben, 2,5 Stunden bis 90°C gerührt und erneut 2,5 g $P_4S_{10}$·Anisol zugegeben und weitere 2 Stunden bei 90°C gerührt. Das Reaktionsgemisch wird filtriert, der Filterrückstand mit Toluol gewaschen und die Toluolphase eingeengt. Der Rückstand wird auf Kieselgel in Cyclohexan EE 1 : 1 chromatographiert.

Ausbeute:

2,5 hellgelbe Kristalle Fp.: 54 - 57°C
(95 % d. Th.) $C_{14}H_{26}N_2SO_2$ MG = 286

NMR:

($CDCl_3$) δppm 60 MHz
0,7-1,8 (m,15H); 2,0 (s,3H) $COCH_3$; 3,05 (s;3H) $N\text{-}CH_3$; 3,0-4,0 (m,3H) $N\text{-}CH_2$, N-CH< ; 4,6-5,1 (m,1H)-CH-OAc; 5,7-6,0 (breites s,1H) CSNH;

Rf-Wert:
Essigester 0,81

Beispiel 13

1-Methyl-2-thioxo-5-(3-hydroxy-octanyl)-imidazolidin XII

0,1 g (0,35 mMol) 1-Methyl-2-thioxo-5-(3-acetoxy-octanyl)-imidazolidin (Beispiel 12) werden in 5 ml MeOH abs. gelöst und mit 100 mg $K_2CO_3$ gepulvert versetzt. Man rührt 3 Stunden bei Raumtemperatur.
Nach beendeter Reaktion wird das Reaktionsgemisch mit 2N Essigsäure auf pH = 6 gestellt und eingeengt. Der Rückstand wird in EE aufgenommen und mit $H_2$ extrahiert. Die getrocknete ($MgSO_4$) EE-Phase wird eingeengt.

Ausbeute:
84 mg helles Öl
(98,4 % d. Th.) $C_{12}H_{24}N_2SO$ MG = 244

NMR:
($CDCl_3$) δppm 60 MHz
0,7 - 2,0 (m,15H); 2,2-2,6 (breites Signal, 1H) OH; 3,1 (s,3H) $N\text{-}CH_3$; 3,1-4,1 (m,4H) $\text{-}N\text{-}CH_2$, N-CH< , >CH-O;

5,9-6,3 (breites Signal, 1H) CSNH;

Rf-Wert:

Methylenchlorid/Methanol 10:1=0,50

Beispiel 14 a

1-Methyl-2-(1-thia-4-ethoxycarbonyl-butyl)-5-(3-hydroxy-octanyl)-Δ2-imidazolin I

$A = CH_2CH_2$ $R^1 = C_2H_5$ $R^2 = C_5H_{11}$ $n = 1$

75 mg (0,26 mMol) 1-Methyl-2-thioxo-5-(3-hydroxy-octanyl)-imidazolidin (Beispiel 13) werden in 3 ml Diglyme abs. auf 90°C unter Rühren und Feuchtigkeitsausschluß erwärmt und 0,1 ml 4-Brombuttersäureethylester zugegeben. Dann wird 4 Stunden gerührt, und es werden erneut bei 90°C weitere 0,1 ml 4-Brombuttersäureethylester zugegeben und 3 Stunden bei 90°C weitergerührt. Die Reaktionslösung wird in 20 ml EE gelöst und mit $NaHCO_3$-Lösung gewaschen. Die EE-Phase wird mit $MgSO_4$ getrocknet, filtriert und im Vakuum eingeengt.

Rohausbeute: 0,14 g helles Öl
Über Merck-Fertigsäule Größe C/-Kieselgel (Korngr.: 0,063-0,2 mm) mit Laufmittel Methylenchlorid 10/ $CH_3OH$ 1 chromatographiert:

Frakt. 25-40 = 50 mg (3'ß-Epimeres)-- Rf-Wert = 0,22 ($CH_2Cl_2/CH_3OH$ 10:1)

Frakt. 54-120 = 52 mg (3'α-Epimeres)- Rf-Wert = 0,13 ($CH_2Cl_2/CH_3OH$ 10:1)

Fortsetzung Beispiel 14 a

Ausbeute:

$C_{18}H_{34}N_2SO_3$ MG = 358 0,10 g (90,5 % d. Th.) helles Öl

NMR ($CDCl_3$):

δppm Spektren für α- und ß-Epimeres im Rahmen der üblichen Auflösung 60 MHz- $^1H$ identisch. 0,9 (t,3H) $CH_3$; 1,26 (t,3H) $COOCH_2$-$\underline{CH_3}$; 1,2 - 1,85 (m,12H) $-CH_2-$; 2,03 (p,2H)-s-$CH_2$-$\underline{CH_2}$-$CH_2$-$CO_2Et$; 2,45 (t,2H) $CH_2$-$CO_2Et$; 2,75 (s,3H) N-$CH_3$; 3,1 (t,3H)-s-$\underline{CH_2}$-$CH_2$; 3,3-3,95 (m,4H)$\rangle$N-$CH_2$, N-CH$\langle$, $\rangle$CH-O; 4,12 (q,2H) $CO_2\underline{CH_2}CH_3$;

Beispiel 14 b - 14 i

In Analogie zu Beispiel 3 a lassen sich aus den Verbindungen der Beispiele 3 b - 3 i durch Anwendung der in Beispiel 4 und in Beispiel 10 - 14 a gegebenen Vorschriften die Verbindungen 14 b - 14 i herstellen. (Formel I, n = 1, $R^2$ siehe Tabelle).

| Beispiel Nr. 14 | NMR-Daten (ppm) charakteristische Signale | $R^2$= | MS (Molmasse) | Rf-werte Essigester Cyclohexan 1:1 α/β-Epimer | Ausbeute % |
|---|---|---|---|---|---|
| b | δ = 0.9 (s,6H) $-C(CH_3)_2$; 1.15 (t,3H) $-OCH_2CH_3$; 3.3 (s,2H) $-OCH_2$; 3.5 (q,2H) $OCH_2CH_3$; | 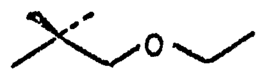 | $C_{19}H_{36}N_2SO_4$ 388.58 | 0.32 / 0.26 | 82 |
| c | δ = 0.9 (d,6H) $C(CH_3)_2$; 7.2 (s,5H) aromat. Prot. | 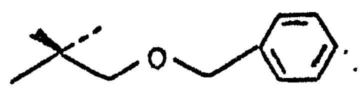 | $C_{24}H_{38}N_2SO_4$ 450.65 | 0.35/0.30 | 86 |
| d | δ = 3.9 (d,2H) $CH_2O$ 6.1-7.3 (dreifacher m, 3H) Thiophen | 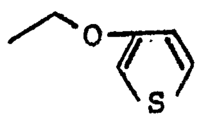 | $C_{18}H_{28}N_2S_2O_4$ 400.57 | 0.45/0.39 | 62 |
| e | δ = 4.95 (m,1H) >CH-F | 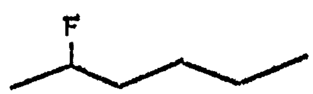 | $C_{18}H_{33}N_2SO_3F$ 376.54 | 0.26/0.19 | 48 |

| Beispiel Nr. 14 | $R^2$= | NMR-Daten (ppm) charakteristische Signale | MS (Molmasse) | Rf-werte $CH_2Cl_2$/MeOH 10 : 1 α/β-Epimer | Ausbeute % |
|---|---|---|---|---|---|
| f | 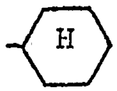 | δ = 1.1– 2.0 (m,11H) CH, $CH_2$; | $C_{18}H_{34}N_2SO_3$ 358.55 | 0.24/0.15 | 92 |
| g | 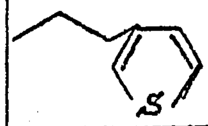 | δ = 6.8–7.3 (m,3H) Thiophen; | $C_{19}H_{30}N_2S_2O_3$ 398.60 | 0.40 / 0.31 | 55 |
| h | 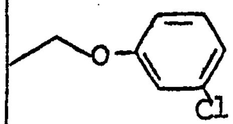 | δ = 3.85 (d,2H) $-CH_2-O-$ 6.0–7.3 (m,4H) aromat. Protonen | $C_{20}H_{29}N_2SO_4Cl$ 428.98 | 0.45/0.34 | 58 |
| i | 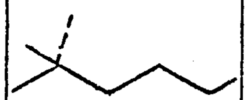 | δ = 0.85 (s, 6H) $-C(CH_3)_2$; | $C_{20}H_{38}N_2SO_3$ 386.61 | 0.3/0.2 | 53 |

Beispiel 15 a

Natriumsalz des 1-Methyl-2-(1-thia-4-carboxy-butyl)-5-(3-hydroxy-octanyl)-Δ2-imidazolin I

(A = $CH_2CH_2$, $R^1$ = Na, $R^2$ = $C_5H_{11}$, n = 1)

358 mg (1 mMol) 1-Methyl-2-(1-thia-4-ethoxycarbonylbutyl)-5-(3-hydroxy-1-octanyl)Δ2-imidazolidin (Beispiel 14 a) werden in 30 ml 80 % Ethanol gelöst. Zu dieser Lösung gibt man unter Rühren eine Lösung von 23 mg Natrium in 4 ml Ethanol/4 ml $H_2O$. Man rührt 3 Stunden bei +10°C unter Argon, filtriert die Lösung über Aktivkohle und entfernt das Lösungsmittel im Vakuum bei 10°C (Gefriertrocknung). Man erhält das Natriumsalz I als farbloses Pulver.

IR-Bande:
KBr-Verreibung-$COO^{\ominus}$ 1610 $cm^{-1}$

Beispiel 15 b

Kaliumsalz des 1-Methyl-2-(1-Thia-4-carboxy-butyl)-5-(3-hydroxy-1-octenyl)-Δ2-imidazolin I

(A = CH=CH, $R^1$ = K, $R^2$ = $C_5H_{11}$, n = 1)

356 mg reines 1-Methyl-2-(1-Thia-4-ethoxycarbonyl-butyl)-5-(3-hydroxy-1-octenyl)-Δ2-imidazolin (Beispiel 9 a), 2 ml 0,5 M Kaliumhydroxidlösung und 2 ml Methanol läßt man unter Inertgas 24 Stunden bei Raumtemperatur stehen. Das Methanol wird im Vakuum abgezogen und die wäßrige Lösung des Kaliumsalzes gefriergetrocknet. Man erhält das Kaliumsalz I als farbloses Pulver.

IR-Bande:
KBr-Verreibung $-COO^{\ominus}$ 1605 $cm^{-1}$

Beispiel 15 c

Triethylammoniumsalz des 1-Methyl-2-(1-Thia-4-carboxy-butyl)-5-(3-hydroxy-1-octenyl)-Δ2-imidazolins I

(n = 1, $R^1 = HN^{\oplus}(C_2H_5)_3$, $R^2 = C_5H_{11}$, A = CH=CH)

Eine wäßrige Lösung von 50 mg des Kaliumsalzes von Beispiel 15 b wird auf eine Säule mit 15 g ® Amberlite CG-50 (Triethylammonium-Form) gegeben. Man eluiert mit einer 3 %igen wäßrigen Lösung von Triethyl-ammoniumcarbonat. Durch Gefriertrocknen des Eluats erhält man das Produkt als kristallines Pulver IR-Bande: KBr-Verreibung $-COO^{\ominus}$ 1600 $cm^{-1}$ (Zers. 85 °C).

In Analogie zu den Beispielen 15 a bis 15 c lassen sich aus den Verbindungen der Beispiele 9 b - 9 i bzw. 14 b - 14 i durch alkalische Esterverseifung und ggf. Chromatographie an Ionenaustauschern die entsprechenden Alkali- oder Ammoniumsalze herstellen.

Beispiel 16

1-Methyl-2-(1-Thia-4-isopropyloxycarbonyl-butyl)-5 (3-hydroxy-4,4-dimethyl-1-octenyl)-Δ1-pyrrolin I

(n = 1, $R^1 = CH(CH_3)_2$ $R^2 = -C(CH_3)-C_4H_9-n$,
A = CH = CH)

356 mg (1 mMol) 1-Methyl-2-(1-Thia-4-ethoxycarbonyl-butyl)-5-(3-hydroxyl-1-octenyl)-Δ2-imidazolin (Beispiel 9 a) werden in 40 ml Isopropanol abs. gelöst und mit 50 mg gepulvertem und gut getrocknetem Kaliumcarbonat

versetzt. Es wird eine Stunde gerührt. Das Lösungsmittel wird im Vakuum abgezogen, der Rückstand in Essigester aufgenommen, EE mit Wasser gewaschen, getrocknet, im Vakuum Lösungsmittel entfernt.

Ausbeute:

345 mg helles Öl
3'ß-Epimeres Rf-Wert Cyclohexan/EE 1:1 = 0.47
3'α-Epimeres Rf-Wert Cyclohexan/EE 1:1 = 0.46

NMR ($CDCl_3$):

δ ppm 4,95 (Septett,1H) <u>CH</u>$(CH_3)_2$; 1,2 (d,6H) $CH(\underline{CH}_3)_2$.

Patentansprüche:

1. Verbindungen der Formel I

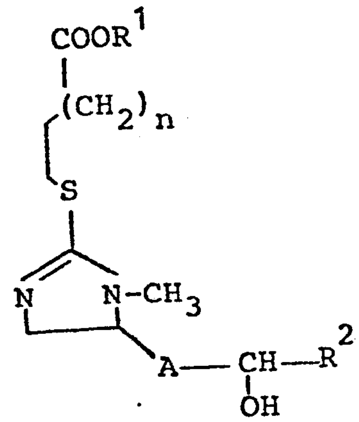

worin bedeuten

$R^1$ Wasserstoff, einen geradkettigen oder verzweigten Alkylrest mit bis zu 8 Kohlenstoffatomen, einen geradkettigen oder verzweigten ungesättigten aliphatischen Kohlenwasserstoffrest mit 3 - 6 Kohlenstoffatomen, einen cycloaliphatischen Kohlenwasserstoffrest mit 3 - 7 Kohlenstoffatomen, einen araliphatischen Kohlenwasserstoffrest mit 7 - 9 Kohlenstoffatomen oder ein physiologisch verträgliches Metall-, $NH_4$- oder ein Ammoniumion, das sich von einem primären, sekundären oder tertiären Amin ableitet, oder ein Tetraalkylammonium,

$R^2$ einen Phenylrest, der im Kern 1 - 3 fach substituiert sein kann mit Halogen, Trifluormethyl und/oder Alkyl oder Alkoxy mit je 1 - 6 C-Atomen, einen cycloaliphatischen Rest mit 3 - 8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 - 8 Kohlenwasserstoffatomen, wobei die aliphatischen Reste ihrerseits substituiert sein können mit

a) einem geradkettigen oder verzweigten Alkoxyrest mit bis zu 6 Kohlenstoffatomen oder einem geradkettigen oder verzweigten Alkenyloxy- oder Alkinyloxyrest mit 3 - 6 Kohlenstoffatomen,

b) Halogen, Cycloalkyl mit 3 - 7 C-Atomen, einem unsubstituierten Phenyl-, α- oder ß-Thienyl- oder α- oder ß-Furylrest, oder einem Phenyl-, Thienyl- oder Furylrest, welche ihrerseits im Kern 1-3 fach substituiert sind mit Halogen, Trifluormethyl und/oder Alkyl oder Alkoxy mit je 1 - 6 C-Atomen,

c) einem unsubstituierten Phenoxy-, α- oder ß-Thienyloxy- oder einem Cycloalkoxyrest mit 3 - 7 Kohlenstoffatomen, oder einem der genannten Reste, welcher im Ring 1 - 3 fach substituiert ist mit Halogen, Trifluormethyl und/oder Alkyloxy mit je 1 - 6 C-Atomen,

A eine $-CH{=}CH$ oder $-CH_2-CH_2$-Gruppe, und

n die Zahl 0, 1, 2, 3 oder 4.

2. Verbindungen der Formel I gemäß Anspruch 1 worin $R^1$ Wasserstoff, $C_1$-$C_4$-Alkyl, $C_2$-$C_4$-Alkenyl, $C_5$-$C_7$-Cycloalkyl, Phenethyl, Benzyl oder ein Ammoniumion, das sich von einem primären, sekundären oder tertiären Amin ableitet, bedeutet,
$R^2$ unsubstituiertes Phenyl oder mit Halogen, Trifluormethyl, $C_1$-$C_4$-Alkyl oder $C_1$-$C_4$-Alkoxy monosubstitu-

iertes Phenyl, unsubstituiertes $C_3$-$C_7$-Alkyl oder $C_3$-$C_7$-Alkyl, das mit $C_5$-$C_7$-Cycloalkyl, $C_1$-$C_3$-Alkoxy, Phenoxy, Halogenphenoxy, Thienyloxy, Halogenthienyloxy, Cyclohexyloxy, Thienyl, Halogenthienyl oder Furyl substituiert ist, bedeutet,
A eine -CH=CH- oder $-CH_2-CH_2-$Gruppe und n die Zahl 1 oder 2 darstellt.

3. Verbindungen der Formel VI

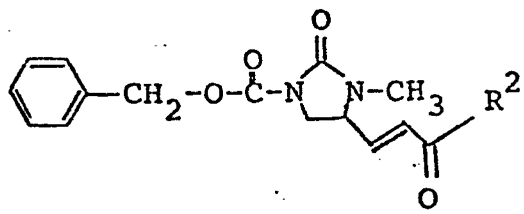

VI

worin $R^2$ die zur Formel I in Anspruch 1 genannten Bedeutungen hat.

4. Verbindungen der Formel

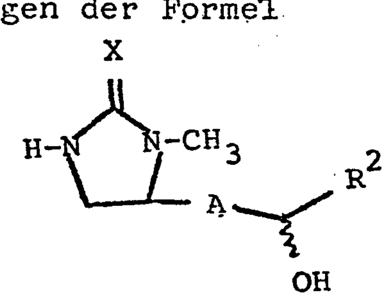

worin X ein Sauerstoff- oder Schwefelatom, A die Gruppe $-CH_2-CH_2-$ oder -CH=CH- darstellt und $R^2$ die zur Formel I in Anspruch 1 genannten Bedeutungen hat.

5. Arzneimittel gekennzeichnet durch den Gehalt an einer Verbindung der Formel I gemäß Anspruch 1 in Mischung mit einem pharmazeutisch üblichen Träger und/oder Stabilisator.

6. Verbindungen der Formel I zur Verwendung als Arzneimittel.

7. Verfahren zur Herstellung von Verbindungen der Formel I

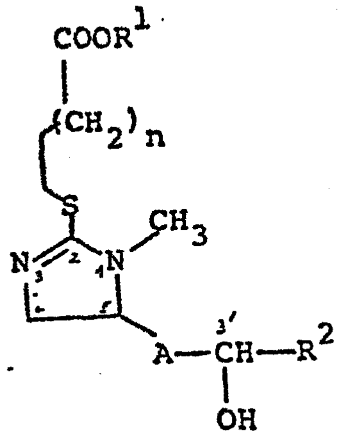

I

worin bedeuten

$R^1$ Wasserstoff, einen geradkettigen oder verzweigten Alkylrest mit bis zu 8 Kohlenstoffatomen, einen geradkettigen oder verzweigten ungesättigten aliphatischen Kohlenwasserstoffrest mit 3 - 6 Kohlenstoffatomen, einen cycloaliphatischen Kohlenwasserstoffrest mit 3 - 7 Kohlenstoffatomen, einen araliphatischen Kohlenwasserstoffrest mit 7 - 9 Kohlenstoffatomen oder ein physiologisch verträgliches Metall-, $NH_4$- oder ein Ammoniumion, das sich von einem primären, sekundären oder tertiären Amin ableitet, oder ein Tetraalkylammonium,

$R^2$ einen Phenylrest, der im Kern 1 - 3 fach substituiert sein kann mit Halogen, Trifluormethyl und/oder Alkyl oder Alkoxy mit je 1 - 6 C-Atomen, einen cycloaliphatischen Rest mit 3 - 8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 - 8 Kohlenwasserstoffatomen, wobei die aliphatischen Reste ihrerseits substituiert sein können mit

a) einem geradkettigen oder verzweigten Alkoxyrest mit bis zu 6 Kohlenstoffatomen oder einem geradkettigen oder verzweigten Alkenyloxy- oder Alkinyloxyrest mit 3 - 6 Kohlenstoffatomen,

b) Halogen, Cycloalkyl mit 3 - 7 C-Atomen, einem unsubstituierten Phenyl-, α- oder ß-Thienyl- oder α- oder ß-Furylrest, oder einem Phenyl-, Thienyl- oder Furylrest, welche ihrerseits im Kern 1-3 fach substituiert sind mit Halogen, Trifluormethyl und/oder Alkyl oder Alkoxy mit je 1 - 6 C-Atomen,

c) einem unsubstituierten Phenoxy-, α- oder ß-Thienyloxy- oder einem Cycloalkoxyrest mit 3 - 7 Kohlenstoffatomen, oder einem der genannten Reste, welcher im Ring 1 - 3 fach substituiert ist mit Halogen, Trifluormethyl und/oder Alkyloxy mit je 1 - 6 C-Atomen,

A eine $-CH=CH$ oder $-CH_2-CH_2$-Gruppe, und

n die Zahl 0, 1, 2, 3 oder 4,

dadurch gekennzeichnet, daß man eine Verbindung der Formel XII

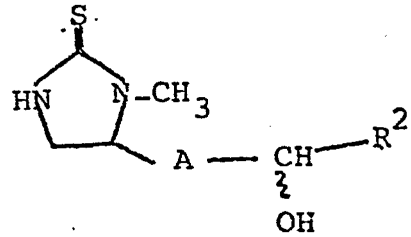

XII

worin A und $R^2$ die obengenannten Bedeutungen haben, umsetzt mit einer Verbindung der Formel

$$Hal-CH_2-CH_2-(CH_2)_n-COOR^1 \quad XIII$$

worin $R^1$ und n die oben genannte Bedeutung haben und Hal, Chlor, Brom oder Jod bedeutet, gegebenenfalls die erhaltene Verbindung der Formel I worin $R^1$ nicht Wasserstoff oder ein Kation bedeutet, verseift zu einer Verbindung der Formel I, worin $R^1$ Wasserstoff oder ein physiologisch verträgliches Kation bedeutet, gegebenenfalls in einer Verbindung der Formel I, worin $R^1$ gleich Wasserstoff oder ein physiologisch verträgliches Metall-, $NH_4$- oder Ammoniumion bedeutet, das sich von einem sekundären, primären oder tertiären Amin ableitet, und $R^2$ und n die zur Formel I gegebenen Bedeutungen haben, das Kation $R^1$ gegen ein anderes ausgetauscht, und gegebenenfalls das erhaltene 3'-Epimerengemisch der Alkohole der Formel I nach üblichen Methoden in das α- und ß-Isomere auftrennt.

8. Verfahren zur Herstellung von Verbindungen der Formel I, dadurch gekennzeichnet, daß man

a) das Imidazolidinon der Formel II

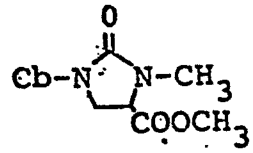 II

worin Cb den Rest

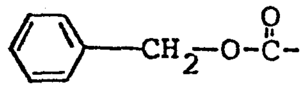

darstellt, durch Reduktion der Esterfunktion in den Imidazolidinonalkohol III überführt,

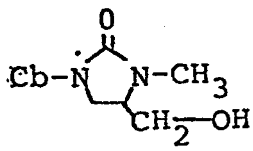

III

b) die Verbindung der Formel III oxyiert zu dem enolisierten Aldehyd der Formel IV

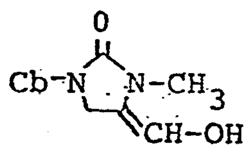

IV

worin Cb die zur Formel II angegebene Bedeutung hat,

c) den Aldehyd der Formel IV umsetzt mit einem Phosphonat der Formel V

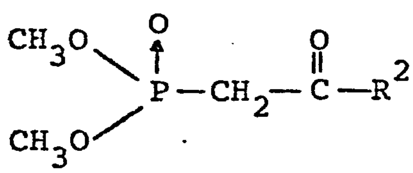

V

worin $R^2$ die zur Formel I angegebene Bedeutung hat, zu einem Enon der Formel VI

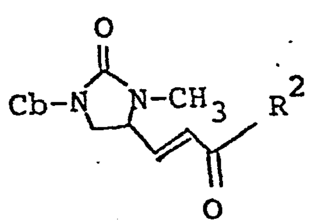

VI

worin $R^2$ die zur Formel I und Cb die zur Formel II angegebene Bedeutung hat,

d) das Enon der Formel VI in bekannter Weise mit einem Reduktionsmittel zu einem 3'-Epimerengemisch der Alkohole der Formel VII reduziert, worin $R^2$ die zur Formel I angegebene Bedeutung hat, und gegebenenfalls das erhaltene 3'-Epimerengemisch der Alkohole der Formel VII nach üblichen Methoden in das α- und ß-Epimere auftrennt,

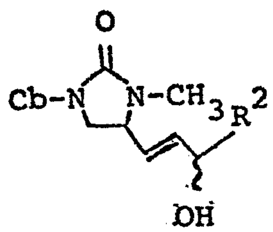

VII

worin $R^2$ die zur Formel I und Cb die zur Formel II angegebene Bedeutung hat,

e) in dem Epimerengemisch der Alkohole der Formel VII oder in den reinen α- bzw. ß-Epimeren die Cb-Schutzgruppe am N-Atom 3 durch alkoholische Hydrolyse abspaltet, wobei man eine Verbindung der Formel VIII

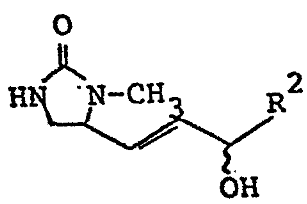

VIII

worin $R^2$ die zur Formel I gegebene Bedeutung hat, erhält, oder

e') das Epimerengemisch der Alkohole oder die reinen α- bzw. ß-Epimeren der Formel VII nach üblichen Methoden hydriert zu einer Verbindung der Formel IX

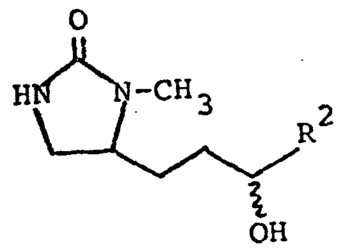

IX

worin $R^2$ die zur Formel I angegebene Bedeutung hat,

f) eine Verbindung der Formel VIII oder IX an der Alkoholfunktion acyliert zu einer Verbindung der Formel X

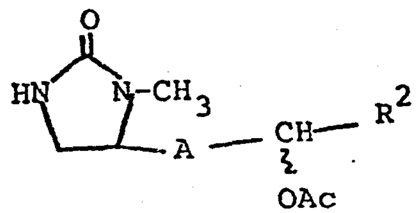

X

worin A eine $-CH=CH-$ oder $-CH_2-CH_2-$Gruppe und Ac einen Alkanoyl-, Arylalkanoyl- oder Cycloalkanoylrest darstellt und $R^2$ die zur Formel I angegebene Bedeutung hat,

g) eine Verbindung der Formel X durch schwefelübertragende Reagenzien nach üblichen Methoden überführt in eine Verbindung der Formel XI

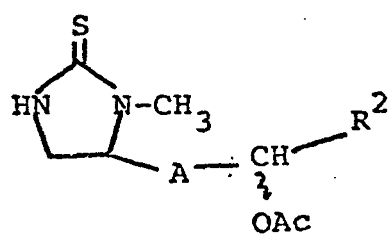

XI

worin A und $R^2$ die zur Formel I und Ac die zur Formel X angegebene Bedeutung haben,

h) in einer Verbindung der Formel XI durch alkalische Hydrolyse die Schutzgruppen Ac abspaltet, wobei man eine Verbindung der Formel XII

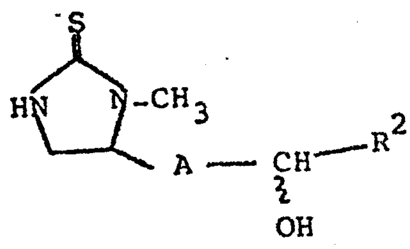

XII

erhält, worin A und $R^2$ die zur Formel I angegebene Bedeutung haben,

i) eine Verbindung der Formel XII mit einer Verbindung der Formel XIII

$$Hal-CH_2-CH_2-(CH_2)_n-COOR^1 \quad XIII$$

worin $R^1$ und n die zur Formel I genannte Bedeutung haben und Hal Chlor, Brom oder Jod bedeutet, umsetzt zu einer Verbindung der Formel I,

k) gegebenenfalls eine Verbindung der Formel I, worin $R_1$ nicht gleich Wasserstoff oder ein Kation ist, verseift zu einer Verbindung der Formel I, worin $R^1$ Wasserstoff oder ein physiologisch verträgliches Kation bedeutet,

l) gegebenenfalls in einer Verbindung der Formel I, worin $R_1$ gleich Wasserstoff oder ein physiologisch verträgliches Metall-, $NH_4$- oder Ammoniumion bedeutet, das sich von einem sekundären, primären oder tertiären Amin ableitet, und $R^2$ und n die zur Formel I angegebenen Bedeutungen haben, das Kation $R^1$ gegen ein anderes ausgetauscht,

m) gegebenenfalls das erhaltene 3'-Epimerengemisch der Alkohole der Formel I nach üblichen Methoden in das α- und ß-Isomere auftrennt.

Europäisches Patentamt

# EUROPÄISCHER RECHERCHENBERICHT

0142027

Nummer der Anmeldung

EP 84112122.1

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | EP - A1 - 0 008 073 (CIRA-GEIGY AG)<br>* Ansprüche 1,10 *<br>---- | 1,5,7 | C 07 D 233/42<br>C 07 D 233/32<br>C 07 D 405/06<br>C 07 D 409/06<br>C 07 D 409/12<br>A 61 K 31/415 |
| | | | RECHERCHIERTE SACHGEBIETE (Int. Cl.4) |
| | | | C 07 D 233/00<br>C 07 D 405/00<br>C 07 D 409/00 |

Der vorliegende Recherchenbericht wurde für alle Patentansprüche erstellt.

| Recherchenort | Abschlußdatum der Recherche | Prüfer |
|---|---|---|
| WIEN | 16-01-1985 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82